# DEMANDE DE BREVET EUROPEEN

(11) **EP 0 876 806 A1**
(43) Date de publication de la demande: **11.11.1998**
(21) Numéro de dépôt: 98401015.7
(22) Date de dépôt: 24.04.1998
(51) Int. Cl.: A61F 2/06

(54) **Extenseurs vasculaires et coronaires, habituellement designes sous le nom de "stent"**

(30) Priorité: 05.05.1997 FR 9705516
(71) Demandeur: Sabaria, Patrick, 78860 Saint nom la Breteche (FR)
(72) Inventeur: Sabaria, Patrick, 78860 Saint nom la Breteche (FR)
(74) Mandataire: Breese, Pierre

(57) **Abrégé**

La présente invention concerne un élargisseur périphérique coronaire ou vasculaire de type " stent " présentant la forme générale d'un élément cylindrique allongé expansible cylindrique et constitué d'une pluralité d'éléments (1, 3, 5) de forme générale annulaire et d'une pluralité de bras de liaison (2, 5) qui interconnectent des éléments annulaires adjacents Cet élargisseur périphérique coronaire ou vasculaire de type " stent " présente une alternance de segments (1, 3, 5) expansibles faiblement flexibles transversalement, et de segments (2, 4) de plus grande flexibilité transversale.

## Description

La présente invention concerne le domaine des extenseurs vasculaires et coronaires, habituellement désignés sous le nom de "stent".

La configuration générale de ces dispositifs est connue dans l'état de la technique, par exemple par le brevet européen EP556850. Ce brevet décrit un stent intraluminal comprenant une longueur déterminée de fil élastique ayant une configuration sinueuse ou en zigzag et formant une hélice continue ayant une série de spires, et une série d'éléments bouclés joignant les sommets voisins des spires d'hélice voisines. Ce stent est compressible et auto-expansible sensiblement jusqu'à une configuration précomprimée.

Un autre brevet européen publié sous le n° EP274846 décrit un élargisseur expansible cylindrique allonge, comportant :
- une première extrémité;
- une seconde extrémité;
- une pluralité d'éléments allongés ayant une section transversale non rectangulaire et s'étendant de la première a la seconde extrémité; et
- une pluralité de noeuds qui interconnectent des éléments adjacents. Les éléments allongés et les noeuds sont formés intégralement d'une seule pièce de matière. L'élargisseur selon ce brevet présente un profil extérieur généralement lisse.

L'inconvénient des stents selon l'état de la technique est que ces stents présente soit un axe de déformation préférentiel selon une direction transversale, soit présente une rigidité transversale importante, ce qui rend difficile l'adaptation à certaines situation présentant des rayons de courbures faibles.

Un autre inconvénient des stents de l'état de la technique est que lors de la dilatation, les stents se déforme légèrement dans la direction transversale.

L'objet de la présente invention est de remédier à ces inconvénients en proposant un stent respectant la courbure de l'artère dans laquelle il est introduit, y compris pendant la dilatation, et garantissant une expansion homogène sur tout sa longueur. Un autre but de l'invention est de proposer un stent dont la courbure est bloquée après dilatation et conserve la configuration qu'il a pu prendre avant dilatation, au moment de l'introduction dans le vaisseau.

A cet effet, l'invention concerne dans son acception la plus générale un élargisseur périphérique coronaire ou vasculaire de type "stent" présentant la forme générale d'un élément cylindrique allongé expansible cylindrique et constitué d'une pluralité d'éléments de forme générale annulaire et d'une pluralité de bras de liaison qui interconnectent des éléments annulaires adjacents caractérisé en ce qu'il présente une alternance de segments expansibles faiblement flexibles transversalement, et de segments de plus grande flexibilité transversale.

Avantageusement, les éléments de faible flexibilité transversale sont constitués par des éléments allongés annulaires réalisés par un fil élastique ayant une configuration sinueuse ou en zigzag dont certaines des boucles sont reliées à certaines des boucles de la configuration sinueuse ou en zigzag de l'élément allongés consécutif par des bras sensiblement longitudinaux, lesdits bras formant les segments de plus grande flexibilité.

Selon une variante préférée, les segments de plus grande flexibilité sont constitués de bras sensiblement longitudinaux et présentant un rapport matière/vide inférieur au rapport matière/vide des segments de moindre flexibilité. Avantageusement, les bras présente une forme de "S". Selon un mode de mise en oeuvre particulier, l'axe médian des bras est incliné par rapport à l'axe longitudinal d'un angle compris entre 0 et ±45°.

Selon une autre variante avantageuse, les bras d'un segment de plus forte flexibilité sont inclinés d'un angle opposé aux bras des segments de plus forte flexibilité avoisinants.

De préférence, le stent selon l'invention est constitué par une alternance d'éléments annulaires formés par une configuration sinueuse présentant une alternance de boucles liées à la boucle de l'élément consécutif par un bras de liaison, et de boucles libres.

Selon un mode de réalisation préféré, le stent est constitué par une alternance d'éléments annulaires formés par une configuration sinueuse présentant en alternance un segment sensiblement longitudinal dont les deux extrémités sont prolongées par des bras de liaison avec les éléments annulaires consécutifs, et 2N-1 segments libres, sensiblement longitudinal, N étant un nombre entier, de préférence égal à 2.

L'invention sera mieux comprise à la lecture de la description d'un exemple non limitatif de réalisation qui suit, se référant aux dessins annexés où
- la figure 1 représente une vue en perspective du stent avant dilatation ;
- la figure 2 représente une vue en perspective du stent après dilatation ;
- la figure 3 représente une vue développée du stent.

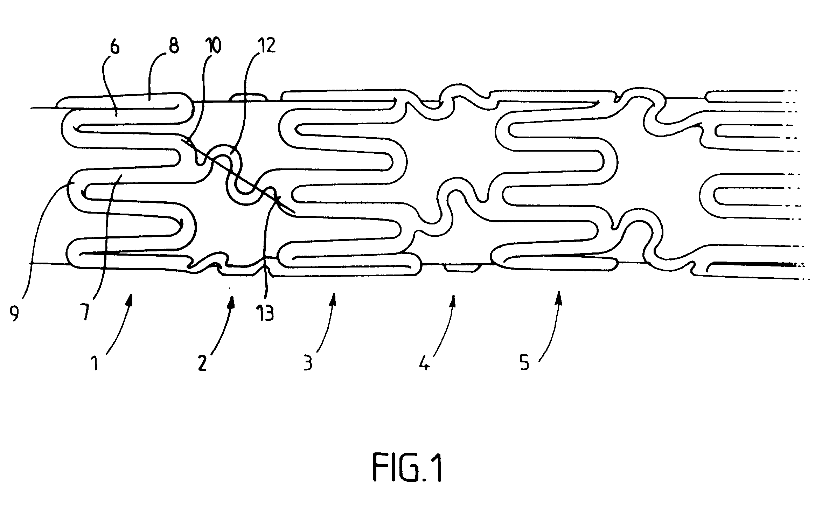

La figure 1 représente une vue en perspective du stent avant dilatation. Il est formé d'une alternance d'éléments de forme extérieure annulaire (1, 3, 5) et d'éléments de grande flexibilité (2, 4).

Les éléments annulaires (1, 3, 5) sont formés par une configuration sinueuse refermée sur elle en forme de couronne. Cette configuration sinueuse est réalisée en acier de type 316 L sans revêtement ni surfaçage. Elle présente une succession des segments (6, 7, 8) reliés par des parties courbes (9, 13, 10) formant un angle de 180°. Ces éléments annulaires présentent une rigidité transversale relativement importante. Ils autorisent par contre une dilatation de l'ordre de 100%, pour passer d'un diamètre initial de 3 mm à un diamètre de 6 mm après dilatation.

Deux éléments annulaires consécutifs (1, 3) sont reliés par des éléments de grande flexibilité formés par une série de bras (12) en forme de "S", dont l'une des extrémités prolonge l'une des boucles (13) du premier élément (1) et dont l'autre extrémité prolonge la boucle (14) de l'élément consécutif (3) faisant sensiblement face à la première boucle (13). En position initiale, l'axe médian (15) du bras (12) forme un angle de l'ordre de 45° avec l'axe longitudinal médian du stent.

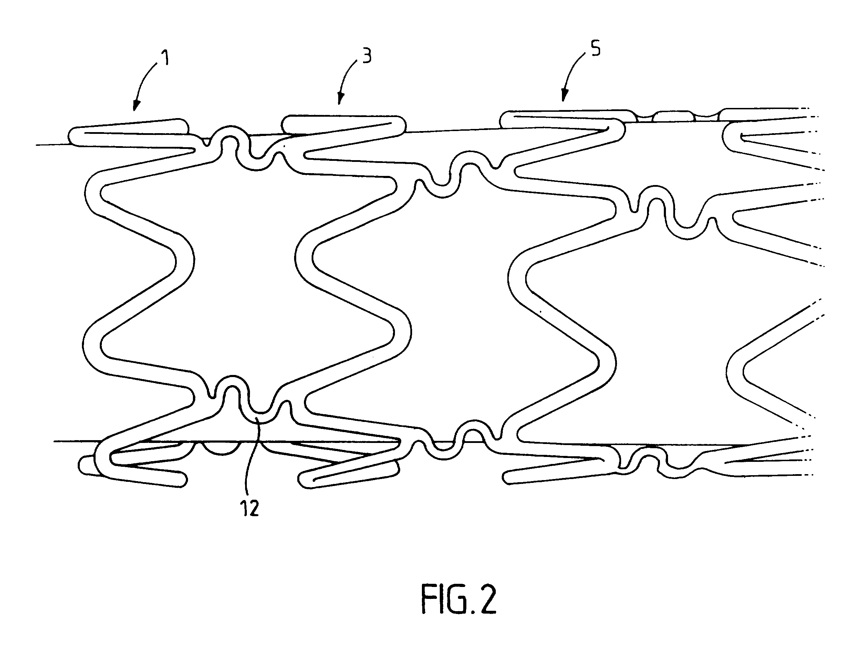

Après dilatation, comme représenté en figure 2, les éléments annulaires (1, 3, 5) se dilatent, ce qui entraînent un pivotement des bras (12) dont l'axe médian devient sensiblement parallèle à l'axe longitudinal du stent.

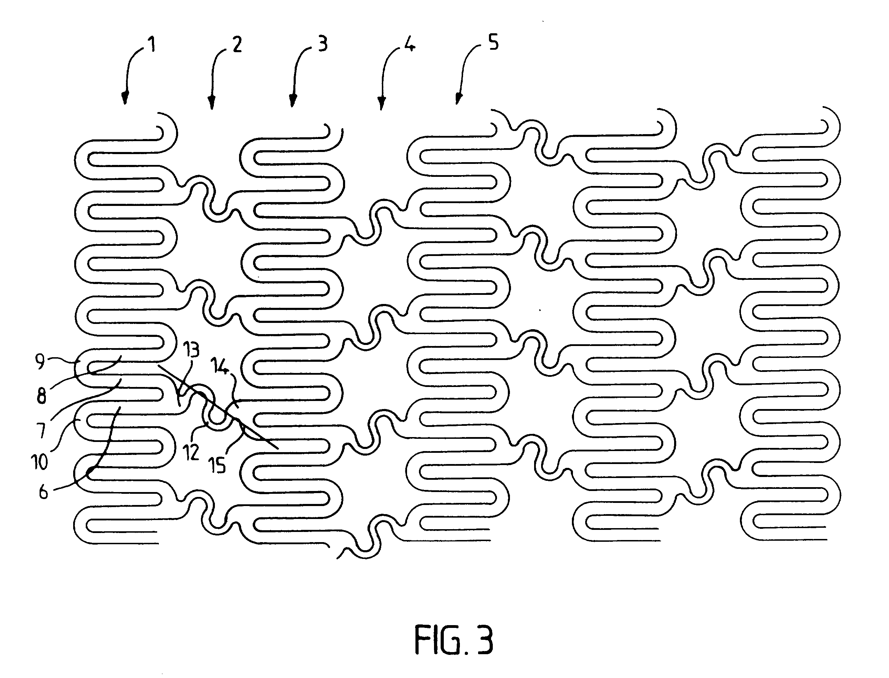

La figure 3 représente une vue développée du stent selon l'invention. A titre d'exemple, la longueur du stent est de 9,25 mm et le périmètre de 5,1 mm, ce qui correspond à un section avant dilatation de 1,6 mm. Le rapport matière/vide des éléments annulaires (1, 3, 5) est de l'ordre de 1 en position non dilatée, et de l'ordre de 0,5 en position dilatée.

Par contre, les éléments de flexibilité (2, 4) présente un rapport matière/vide très inférieur, de l'ordre de 0,1. Les bras (12) se raccordent sensiblement tangentiellement aux boucles. Ils présentent une forme générale de "S", formée de deux segments semi-annulaires reliés tête-bêche. Seule une boucle sur deux est prolongée par un bras. La boucle intercalaire est libre.

Deux éléments flexibles consécutifs (2, 4) présentent des bras dont les axes médians présentent des inclinaisons opposées. Ceci garantit une homogénéité de la dilatation.

L'invention est décrite dans ce qui précède à titre d'exemple non limitatif. Il est entendu que l'homme du métier sera à même de réaliser diverses variantes sans pour autant sortir du cadre de l'invention.

## Revendications

1. Elargisseur périphérique coronaire ou vasculaire de type "stent" présentant la forme générale d'un élément cylindrique allongé expansible cylindrique et constitué d'une pluralité d'éléments (1, 3, 5) de forme générale annulaire et d'une pluralité (2, 4) de bras de liaison (12) qui interconnectent des éléments annulaires (1, 3, 5) adjacents caractérisé en ce qu'il présente une alternance de segments expansibles (1, 3, 5) faiblement flexibles transversalement, et de segments (2, 4) de plus grande flexibilité transversale.

2. Elargisseur périphérique coronaire ou vasculaire selon la revendication 1 caractérisé en ce que les éléments (1, 3, 5) de faible flexibilité transversale sont constitués par des éléments allongés annulaires réalisés par un fil élastique ayant une configuration sinueuse ou en zigzag dont certaines des boucles (13) sont reliées à certaines des boucles de la configuration sinueuse ou en zigzag de l'élément allongés consécutif par des bras sensiblement longitudinaux, lesdits bras (12) formant les segments de plus grande flexibilité.

3. Elargisseur périphérique coronaire ou vasculaire selon la revendication 1 ou 2 caractérisé en ce que les segments de plus grande flexibilité sont constitués de bras (12) sensiblement longitudinaux et présentant un rapport matière/vide (1, 3, 5) inférieur au rapport matière/vide des segments de moindre flexibilité.

4. Elargisseur périphérique coronaire ou vasculaire selon la revendication 2 ou 3 caractérisé en ce que les bras (12) présentent une forme de "S".

5. Elargisseur périphérique coronaire ou vasculaire selon la revendication 2, 3 ou 4 caractérisé en ce que l'axe médian des bras (12) est incliné par rapport à l'axe longitudinal d'un angle compris entre 0 et ±45°.

6. Elargisseur périphérique coronaire ou vasculaire selon la revendication 5 caractérisé en ce que les bras (12) d'un segment (2) de plus forte flexibilité sont inclinés d'un angle opposé aux bras des segments (4) de plus forte flexibilité avoisinants.

7. Elargisseur périphérique coronaire ou vasculaire selon la revendication 1 caractérisé en ce qu'il est constitué par une alternance d'éléments annulaires (1, 3, 5) formés par une configuration sinueuse présentant une alternance de boucles (13) d'un premier élément annulaire (1) liées à la boucle (15) de l'élément consécutif (3) par un bras de liaison (12), et de boucles libres.

8. Elargisseur périphérique coronaire ou vasculaire selon la revendication 1 caractérisé en ce qu'il est constitué par une alternance d'éléments annulaires (1, 3, 5) formés par une configuration sinueuse présentant en alternance un segment (6) sensiblement longitudinal dont les deux extrémités (10, 13) sont prolongées par des bras de liaison (12) avec les éléments annulaires consécutifs, et 2N-1 segments libres, sensiblement longitudinal, N étant un nombre entier.

9. Elargisseur périphérique coronaire ou vasculaire selon la revendication 8 caractérisé en ce que N est égal à 2.

10. Elargisseur périphérique coronaire ou vasculaire selon la revendication 7 caractérisé en ce qu'il est constitué par une alternance d'éléments annulaires formés chacun par une configuration sinueuse présentant en alternance un segment sensiblement longitudinal dont, pour chacune des extrémités, une boucle sur deux est reliée par un bras oblique à une boucle de l'élément annulaire adjacent, et une boucle sur deux est libre.
